# EUROPÄISCHE PATENTSCHRIFT

(11) **EP 0 667 917 B1**
(45) Veröffentlichungstag und Bekanntmachung des Hinweises auf die Patenterteilung: **09.04.1997**
(21) Anmeldenummer: 93924581.7
(22) Anmeldetag: 03.11.1993
(51) Int. Cl.: C12Q 1/18, A61K 39/00, A61K 39/395

(54) **EVOLUTIVE VAKZINIERUNG**
EVOLUTIVE VACCINATION
VACCINATION EVOLUTIVE

(30) Priorität: 05.11.1992 DE 4237382; 25.02.1993 DE 4305736
(43) Veröffentlichungstag der Anmeldung: 23.08.1995
(73) Patentinhaber: EVOTEC BIOSYSTEMS GMBH, D-22529 Hamburg (DE)
(72) Erfinder: HENCO, Karsten, D-40699 Erkrath (DE); EIGEN, Manfred, D-37075 Göttingen (DE); LINDEMANN, Björn, D-37085 Göttingen (DE); SCHWIENHORST, Andreas, D-37075 Göttingen (DE)
(74) Vertreter: Meyers, Hans-Wilhelm, Dr.Dipl.-Chem.
(86) Internationale Anmeldenummer: EP9303076
(87) Internationale Veröffentlichungsnummer: WO9410339

(56) Entgegenhaltungen:
- WO-A-92/18616
- DATABASE WPI Week 8847, Derwent Publications Ltd., London, GB; AN 88-338051 HIZI ET AL 'HUMAN IMMUNO-DEFICIENCY VIRUS REVERSE TRANSCRIPTASE VARIANT-USEFUL IN RECOMBINANT ESCHERICHIA COLI SYSTEM ROF SCREENING DRUGS DESIGNED TO INHIBIT VIRAL REPLICATION' & US,A,7 110 348 (US DEPT HEALTH & HUMAN) 11. Oktober 1988
- FILE SERVER STN KARLSRUHE,FILE MEDLINE ZUSAMMENFASSUNG NR. 89259022 & J VIROL, (1989 JUN) 63 (6) 2476-85
- FILE SERVER STN KARLSRUHE,FILE BIOSIS ZUSAMMENFASSUNG NR. 91:475296 & J VIROL 65 (9).1991.4777-4785
- FILE SERVER STN KARLSRUHE,FILE MEDLINE ZUSAMMENFASSUNG NR. 91056547 & J VIROL, (1990 DEC) 64 (12) 5797-803
- FILE SERVER STN KARLSRUHE,FILE MEDLINE ZUSAMMENFASSUNG NR. 90211672 & AIDS, (1989 DEC) 3 (12) 777-84
- NUCLEIC ACIDS RESEARCH Bd. 20, Nr. 17 , 11. September 1992 , LONDON,GB Seiten 4581 - 4589 CHEN ET AL 'MULTITARGET-RIBOZYME DIRECTED TO CLEAVE AT UP TO NINE HIGHLY CONSERVED HIV-1 ENV RNA REGIONS INHIBITS HIV-1 REPLICATION-POTENTIAL EFFECTIVENESS AGAINST MOST PRESENTLY SEQUENCED HIV-1 ISOLATES'
- FILE SERVER STN KARLSRUHE,FILE MEDLINE ZUSAMMENFASSUNG NR. 92123720 & PROC R SOC LOND (BIOL), (1991 NOV 22) 246 (1316) 141-6
- DATABASE WPI Week 9318, Derwent Publications Ltd., London, GB; AN 93-152045 & US,A,90 688 192 (US DEPT HEALTH & HUMAN SERVICE) 02 Juli 1992 LISZIEWICZ ET AL 'CELL CULTURE SYSTEM FOR EVALUATING ANTIVIRAL DRUG TREATMENT-COMPRISES LONG TERM CULTURE WHICH ALLOWS PRODN. OF ESCAPE MUTANTS'
- PROCEEDINGS OF THE NATIONAL ACADEMY OF SCIENCES,USA Bd. 89 , Dezember 1992 , WASHINGTON D.C.,USA Seiten 11209 - 11213 LISZIEWICZ ET AL 'SPECIFIC INHIBITION OF HUMAN IMMUNODEFICIENCY VIRUS TYPE 1 REPLICATION BY ANTISENSE OLIGONUCLEOTIDES: AN IN VITRO MODEL FOR TREATMENT''

## Beschreibung

Gegenstand der vorliegenden Erfindung ist eine Substanz oder ein Substanzgemisch gegen pathogene Organismen, Arzneimittel gegen pathogene Organismen sowie Screening-Verfahren zur Identifizierung von Wirkstoffen gegen die pathogenen Organismen, die in den Arzneimitteln gemäß der Erfindung Verwendung finden.

Bei einer Infektion eines Organismus mit pathogenen Organismen wie Viren oder Bakterien erzeugt das Immunsystem, durch Antwort auf diese Infektion, Fluchtmutanten dieser pathogenen Organismen, die wiederum eine Immunantwort auslösen, wodurch eine weitere Generation Fluchtmutanten selektiert wird usw. Bei der medikamentösen Therapie gegen Infektionen mit pathogenen Organismen stellen sich meistens auch sehr schnell Resistenzphänomene ein, d.h. der zunächst wirksame Stoff gegen die pathogenen Organismen verliert seine Wirksamkeit, da sich resistente pathogene Organismen bilden, die gegen den verabreichten Wirkstoff resistent sind. So zeichnen sich Viren durch eine große Variabilität ihrer Genome aus. Diese beruht auf einer fehlerhaften Replikation durch Virusspezifische Polymerasen. M. Eigen und andere Forschergruppen konnten zeigen, daß Viren dicht an ihrer theoretischen Fehlerschwelle repliziert werden.

Eine Replikation oberhalb dieser Schwelle würde dafür sorgen, daß die Information der Viren irreversibel verlorenginge. Eine Replikation dicht unterhalb dieser Schwelle verleiht dem Genom eine maximale Plastizität und verleiht einem Virus die Fähigkeit auf evolutiven Streß rasch zu reagieren. So können sich beispielsweise beim Angriff eines antiviralen Wirkstoffes auf den infektiösen Virus solche Mutanten schnell durchsetzen, deren Mutation dem Virus eine mehr oder weniger gute Anpassung an die neue Situation verleiht.

So läßt sich die durch Polioviren verursachte Kinderlähnung bekanntermaßen zwar durch eine aktive Vakzinierung äußerst erfolgreich kontrollieren, dennoch sind Impfdurchbrüche bei bestimmten Einzelfällen zu beobachten gewesen. Die Vakzinierung wird mit sogenannten attenuierten Stämmen durchgeführt. Diese werden durch in vitro Passagen gewonnen, bis sich der Verlust der Pathogenität einstellt.

Mit den Methoden der Molekularbiologie ist es gelungen, ein Verständnis über die Natur der Vakzine zu erhalten und Erklärungen für die gelegentlichen Impfdurchbrüche zu finden. Einer der eingesetzten attenuierten Stämme zeichnet sich durch lediglich drei Mutationen auf Nukleotidebene aus, von denen überhaupt nur zwei sich auf der Expressionsebene äußern durch entsprechenden Austausch von Aminosäuren in bestimmten Proteinen. Hinzu kommt, daß auch die Polio-Polymerase mit einer bestimmten Fehlerrate Nukleinsäuren repliziert, wenn diese auch nicht ganz so stark mit Fehlern behaftet ist, wie diejenige des HIV. Durch die fehlerhaft replizierende Polymerase werden in kurzer Zeit Revertanten produziert, so daß lediglich zwei Rückmutationen den Wildtyp wiederherstellen können. In den wenigen Fällen der Polio-Impfdurchbrüche ist die Situation gegeben, daß beim zeitpunkt der Revertantenbildung die Immunantwort noch nicht spezifisch und stark genug ausgeprägt ist, um auch die pathogenen Mutanten zu unterdrücken. Wahrscheinlich besteht eine quantitative Schwelle für ein bestimmtes Virussystem, jenseits derer ein Immunsystem die Kontrolle der Virusproliferation verliert.

Trotz des unbestrittenen Erfolges dieser seit langen Jahren zugelassenen Immunisierungsstrategie muß leider festgestellt werden, daß dieser Impfstoff nach heutigen Regeln als rekombinanter Impfstoff zumindest in Deutschland keine Chance auf eine Arzneimittelzulassung hätte. Zukünftige Impfstrategien müssen auch diese Aspekte berücksichtigen.

Das HIV-Virus stellt heutzutage eine scheinbar neue Bedrohung dar. Es gab anfänglich Vermutungen, daß das Virus erst vor wenigen Jahrzehnten entstanden sein könnte. M. Eigen konnte jedoch zeigen, daß die Retroviren der HIV-Gruppe vor mehreren hundert Jahren entstanden sein mußten. Diese Daten lassen sich sicher aus den Sequenzen heutiger Viren bestimmen. Dennoch handelt es sich offensichtlich erst seit den 70er Jahren um ein internationales epidemiologisches Problem. Daran sind unter anderem folgende Faktoren beteiligt:
- hohe Mutationsraten verursacht durch die beteiligten viralen Polymerasen,
- Wirtsspezifität für Immunzellen
- Persistenzeigenschaft
- traditionelle Therapieansätze
verbunden mit neuen sozioökologischen Faktoren.

Offensichtlich ist in nahezu allen Fällen bei einer Infektion mit HIV-Viren die Variabilität des Genoms der Viren so groß, daß trotz einer effektiven Immunantwort ein HIV-Virus nicht dauerhaft kontrolliert oder gar die Infektion kuriert werden kann. Zu viele Mutanten in Form resistenter pathogener Mutanten (Fluchtmutanten) werden generiert und selektiert. Dieser Zyklus hält solange an, bis offensichtlich das immunologische Abwehr-Repertoire des infizierten Organismus erschöpft ist und es dann zu einer ungehinderten Ausbreitung des Virus kommen kann. Eine anti-virale Therapie konventioneller Art verstärkt fatalerweise diesen Effekt. In recht kurzer Zeit (Wochen bis Monate) werden weitere Mutanten selektiert, die zusätzlich Resistenz gegen Wirkstoffe erworben haben, ohne daß man in der Lage ist, die Folgeerscheinungen abzuschätzen. Aufgabe einer wirksamen Therapie ist es somit, diesen Teufelskreis zu durchbrechen und Resistenzbildungsmechanismen gegen das Immunsystem oder gegen Pharmaka zu unterbinden.

Die Patentanmeldung P 42 22 289 schlägt zwar einen Weg zur Verhinderung der Resistenzbildung auf viraler Ebene vor, eine Strategie, den Zusammenbruch der Immunantwort zu verhindern und eine schleichende Resistenzbildung nicht zuzulassen, wird jedoch nicht gegeben.

J. Virol. 65 (1991), S. 4777 - 85 betrifft Varianten von molekular klonierten HIV-1, die analysiert wurden mit dem Merkmal nach Exposition zu löslichen rekombinanten CD4 zu replizieren. Dabei wurden Varianten gefunden, die eine 8-fach bzw. 16-fach reduzierte Empfindlichkeit bezüglich der rekombinanten CD4-Neutralisation aufwiesen gegenüber dem Wildtypvirus.

J. Virol. 64 (1990), S. 5797 - 803 betrifft den Mechanismus antigener Variationen an individuellen Epitopen der Influenza Virus N9 Neuraminidase.

J. Virol. 63 (1989), S. 2476 - 85 offenbart die genetische und molekulare Analyse spontaner Mutanten des menschlichen Rhinovirus 14, die gegenbestimmte antivirale Verbindungen resistent sind.

Nucl. Acids Res 20 (September 1992) Seiten 4581 bis 4589 offenbart die Herstellung von Mono-, Di- Tetra-, Penta- und Nonaribozymen. Diese Multitarget-Ribozyme wurden eingesetzt, um HIV-1 envRNA an bis zu 9 verschiedenen konservierten Stellen zu spalten.

In PNAS Vol. 89 (Dezember 1992), Seiten 11209 bis 11215 wird die Entwicklung eines Kultursystems beschrieben, das in vivo-Bedingungen einer HIV-1-Infektion simuliert, um Langzeitwirkungsstudien mit Antisense-Oligonucleotiden durchzuführen. Dabei trat im Falle der Inhibition der HIV-Replikation mit einem Splice-Acceptor Site Antisense-Oligodeoxynucleotid nach 25 Tagen Behandlungszeit eine Fluchtmutante auf. Dies wurde unterdrückt, wenn die Antisense-Oligodeoxynucleotide komplementär zu Sequenzabschnitten des REV-Responsive-Elementes und REV-1-Genes waren.

Der vorliegenden Erfindung liegt das technische Problem zugrunde, das evolutive Verhalten viraler Quasi Spezies-Verteilungen und ihr Vermögen sich rasch antiviralen Selektionsdrücken anzupassen, auf Seiten des Immunsystems zu unterbrechen. Es sollen mithin Stoffe, Arzneimittel und Verfahren bereitgestellt werden, die einen Zusammenbruch der Immunantwort verhindern, aufgrund einer schleichenden Resistenzbildung durch äußeren Selektionsdruck auf die infektionen, pathogenen Organismen bzw. deren Fluchtmutanten.

Das der Erfindung zugrundeliegende Problem wird gelöst durch eine Substanz oder ein Substanzgemisch mit den Merkmalen des Anspruchs 1. Die Unteransprüche 2 bis 6 betreffen bevorzugte Ausführungsformen der Substanz oder des Substanzgemisches.

Anspruch 7 betrifft ein Screening-Verfahren zur Identifizierung von wirksamen Substanzen.

Anspruch 8 betrifft ein Arzneimittel enthaltend eine Kombination aus einem Wirkstoff gegen den betreffenden pathogenen Organismus und Wirkstoffen gegen die Fluchtmutanten dieses pathogenen Organismus.

Die erfindungsgemäßen Substanzen oder Substanzgemische sind erhältlich durch die Züchtung von pathogenen Organismen einer bestimmten Sorte in Gegenwart eines Wirkstoffes gegen diese pathogenen Organismen. Dabei wird bewußt die Bildung von gegen diesen Wirkstoff resistenten, pathogenen Organismen (Fluchtmutanten) erwartet. Die Fluchtmutanten dienen dann als Ausgangspunkt für die Herstellung von Antiseren in an sich bekannter Weise gegen die resistenten pathogenen Organismen (Fluchtmutanten). Es können jedoch ebenso die Epitope funktionaler Elemente der resistenten pathogenen Organismen (Fluchtmutanten) zur Gewinnung von Antiseren verwendet werden.

Es kann vorteilhaft sein, das Verfahren zur Gewinnung der Substanzen oder Substanzgemische mehrfach zu durchlaufen, wobei jeweils wiederum wirkstoffe gegen die Fluchtmutanten eingesetzt werden, um Fluchtmutanten zweiter Generation zu bilden und zu erfassen. Es ist dann vorteilhaft, die im Schritt c) gewonnenen Antiseren entweder als Wirkstoff für Schritt a) zu verwenden oder anstelle eines Antiserums auch einen anderen Wirkstoff, der gegen die Fluchtmutante wirkt, als Wirkstoff zu verwenden, in dem die Fluchtmutante gezüchtet wird.

Die Züchtung der pathogenen Organismen oder der Fluchtmutanten kann in vitro in Zellen in einem Flußreaktor erfolgen. Das Auftreten resistenter Mutanten der pathogenen Organismen kann beispielsweise durch Messung der lytischen Aktivität dieser pathogenen Organismen oder deren Fluchtmutanten erfolgen. Dabei kann beispielsweise der Zell-Stat als Flußreaktor dienen. Der Zell-Stat besteht z. B. aus einer Vorrichtung aus der kontinuierlich Wirtszellen in einen Reaktor gespeist werden, der gleichzeitig einen Abfluß besitzt. Die Zahl intakter Zellen kann turbidometrisch bestimmt werden, so daß bei einer fortschreitenden Infektion der Zellen durch ein lysierendes Virus, die Zahl der Zellen über die Regelung des Zuflußes kontrolliert werden kann. Die Infektion von Zellen durch Viren wird mit gleichzeitiger geringer Gabe eines antiviralen Wirkstoffes so eingestellt, daß meßbare aber geringe Lyse der Wirtszellen und somit eine geringe Viruspropagation einhergeht. Sobald sich nur eine resistente Mutante einstellt, zeigt sich eine lytische Aktivität, der mit höherer Wirkstoffdosis begegnet wird. Wenn solche Dosen erreicht worden sind, wie sie maximal auch im Rahmen einer Therapie eingesetzt werden, wird vorzugsweise die erhaltene neue Quasi Spezies analysiert. Charakteristische Mutationen können dabei nicht nur über die Methode der Sequenzierung erhalten werden, sondern auch durch Methoden wie Temperaturgradienten-Gelelektrophorese oder SSCP, mit denen sich mutierte Bereiche schnell eingrenzen lassen, bevor sie der Sequenzanalyse unterworfen werden.

Mit dieser Methode wurden in bakteriellen Systemen Qβ-Phagen Varianten isoliert, die in der Lage waren, die palindromische Regulationsregion für die RNA-Replikation so zu mutieren, daß sie nicht mehr durch zellulär exprimiertes Coat-Protein blockiert werden konnte.

In einer weiteren bevorzugten Ausführungsform kann die Züchtung der pathogenen Organismen auch durch Wachstum in Gegenwart steigender Konzentration des Wirkstoffes gegen die pathogenen Organismen erfolgen, bis die ersten Mutanten auftreten. Diese Vorgehensweise ist mit der Methode der seriellen Verdünnung vergleichbar, wobei eine Viruspopulation immer wieder verdünnt wird und gegen eine stetig erhöhte Wirkstoffkonzentration anwachsen muß, bis die resistenten Varianten erzeugt werden.

Mit dieser Methode konnte in einem in vitro·Qβ-Phagen abgeleiteten Replikationssystem mit sogenannten Minivarianten ein Paar von (+/-)-Strängen isoliert werden, das mit äußerst hohen Konzentrationen des RNA-spaltenden Enzyms RNAse A verträglich war. RNAse A spaltet RNA-Stränge hinter Pyrimidinen, wenn sich diese in Einzelstrangregionen befinden. Mit einer 18-Fehler (+)-Strang-Mutante fand dieses System innerhalb weniger Passagen ein RNAse A tolerierendes Replikationssystem. An 18 Positionen waren Pyrimidine gegen Purine ausgetauscht. Der (-)-Strang konnte mit den entsprechenden Purinen an diesen Positionen nur überleben, da dessen Konzentration mit ca. 10 % gegenüber dem (+)-Strang 10fach kleiner war, die Schnittstellen für Ribonuklease A immer mit Replikase besetzt waren und von dem der (+)-Strang > 10fach schneller abgelesen wurde als umgekehrt der (-)-Strang von der (+)-Strang Matrize.

Als pathogene Organismen, gegen die Substanzen oder Substanzgemische erhältlich sind, kommen insbesondere Viren, Bakterien, Pilze oder andere Krankheitserreger in Betracht.

Die Antiseren, die sich gegen die Fluchtmutanten bilden, sind beispielsweise gegen Epitope funktionaler Elemente dieser Fluchtmutanten gerichtet. Üblicherweise sind die Epitope funktionaler Elemente der Fluchtmutanten Aminosäuresequenzen.

Das erfindungsgemäße Screening-Verfahren kann zur Identifizierung von wirksamen Substanzen gegen pathogene Organismen herangezogen werden. Dazu werden die pathogenen Organismen in Gegenwart eines Wirkstoffes gezüchtet, wie bereits oben beschrieben wurde, bis hin zum Auftreten von Fluchtmutanten. Diese Fluchtmutanten werden dann als Zielorganismen für die Behandlung mit weiteren Substanzen, die als Wirkstoffe gegen die Fluchtmutanten in Frage kommen, verwendet. Sofern sich gegen die Fluchtmutanten wirksame Substanzen auffinden lassen, können diese als Wirkstoffe gegen die Fluchtmutanten klassifiziert werden.

Das erfindungsgemäße Arzneimittel enthält eine wirksame Menge eines Wirkstoffes gegen die pathogenen Organismen zusammen mit einer oder mehreren Substanzen oder Substanzgemische wie sie in den Ansprüchen 1 bis 6 beschrieben sind und/oder Wirkstoffe, die nach dem erfindungsgemäßen Screening-Verfahren identifiziert wurden neben üblichen pharmazeutischen Hilfs- und Trägerstoffen.

Die erfindungsgemäße antivirale Strategie basiert auf dem Verständnis für das evolutive Verhalten viraler Quasi Spezies, die von M. Eigen (1971) beschrieben wurden und ihrem Vermögen sich rasch antiviralen Selektionsdrücken anzupassen. Das erfindungsgemäße Verfahren betrachtet nicht isoliert die immunologische Therapie einer Wildtyp-Population, sondern den Verbund mit einer vorhersehenden immunologischen Therapie gegen die Fluchtmutanten. Das erfindungsgemäße Verfahren befaßt sich mit dem zeitlichen Antizipieren der Virusmutanten, die durch das Ausweichen des Virus unter dem Therapiedruck entstehen, durch Vorgreifen der Bereitstellung der immunologischen oder medikamentösen Gegenwehr. Dadurch wird die Verzögerung der immunologischen Reaktionszeit des natürlichen Systems aufgehoben. So wird die Evolution eines Virus durch die immunologische Abwehr überholt, da entweder die Immunabwehr die Fluchtmutantenstrukturen bereits kennt oder aber ein Wirkstoff gleich die erzeugten Fluchtmutanten angreift und somit Viren einem schädlichen Einfluß auf den infizierten Organismus entzieht.

Die Zeit der evolutiven Entwicklung ist die Variable, die dafür sorgt, daß zumindest eine Situation wie bei der Polio-Impfung erreicht wird. Die Therapie besteht nun aus einer Kombination traditioneller Therapeutika zur Unterbindung einer Virusreplikation und beispielsweise aktiv immunisierenden Substanzen, die antigene Strukturen noch nicht existenter oder dominierender Fluchtmutanten tragen.

Bei einer Analyse von HIV-Varianten, die Resistenzen gegen den Wirkstoff AZT ausgebildet haben, hat sich gezeigt, daß eine limitierte Anzahl von bis zu fünf Mutationen im Bereich des Polymerase-Gens für eine effiziente Resistenz gegen den verabreichten Wirkstoff ausreichend ist. Die Tatsache, daß immer wieder dieselben Mutanten gefunden werden, die sich unabhängig voneinander entwickeln, deutet darauf hin, daß diese Mutationen einen besonders hohen Selektionsvorteil gegenüber weiteren potentiell AZT-resistenten Mutanten aufweisen, so daß erstere sich letztlich durchsetzen. Damit ist jedoch keinesfalls gesagt, daß nicht weitere Mutanten existieren, die als solche oder Mutanten davon wiederum auch dem neuerlichen evolutiven Streß auszuweichen vermögen. Demnach ist es vorteilhaft eine Wirkstoffkaskade aufzubauen, bis das Erreger-Potential gegenüber dem Immunsystem soweit geschwächt ist, daß es seine Ausweichmöglichkeiten wie im Falle des Polio-Beispiels verliert. Ein besonders vorteilhafter Effekt der Therapie besteht dann darin, daß durch die effiziente Unterdrückung der Fluchtmutanten auch deren mögliche Verbreitung auf weitere Organismen auf ein Minimum beschränkt wird, so daß das Immunsystem Gelegenheit hat, mit der relativ geringen Anzahl der möglicherweise noch vorhandenen Fluchtmutanten selbständig fertig zu werden.

Es wird nun zum Beispiel ein Verfahren sinnvoll, bei dem mit den ersten AZT-Gaben gleichzeitig eine Vakzinierung mit mindestens einem antigenen Epitop von mindestens einer Fluchtmutante durchgeführt wird. Das Verfahren grenzt sich deutlich von anderen Ansätzen von Kombinationstherapien ab, die beabsichtigen, durch gezielten Einsatz mehrerer antiviraler wirkstoffe die Wahrscheinlichkeit einer Resistenzbildung herabzusetzen. Die konventionellen Verfahren zielen einzig und allein darauf ab, daß die Wahrscheinlichkeit der Bildung von Fluchtmutanten mit der Anzahl benötigter Mutationen sinkt, nimmt jedoch die Bildung dieser Fluchtmutanten in Kauf.

Das Beispiel zeigt, wie es mit Hilfe dieses Verfahrens möglich wird, in artifiziellen Modellsystemen die Fluchtbewegungen eines Mikroorganismus zu simulieren und die Mutanten zu ermitteln. Auf diese Weise läßt sich eine erfindungsgemäße Kombination aus Wirkstoffen ermitteln, deren Fähigkeit es ist, der evolutiven Fluchtbewegung eines Mikroorganismus zuvorzukommen, bzw. diese zu antizipieren. Dabei werden die Epitope funktionaler Proteine eines Mikroorganismus, deren Mutanten unter dem Einfluß eines antiviralen Wirkstoffes in Form einer neuen überlegenen Consensus-Sequenz selektiert werden, verwendet.

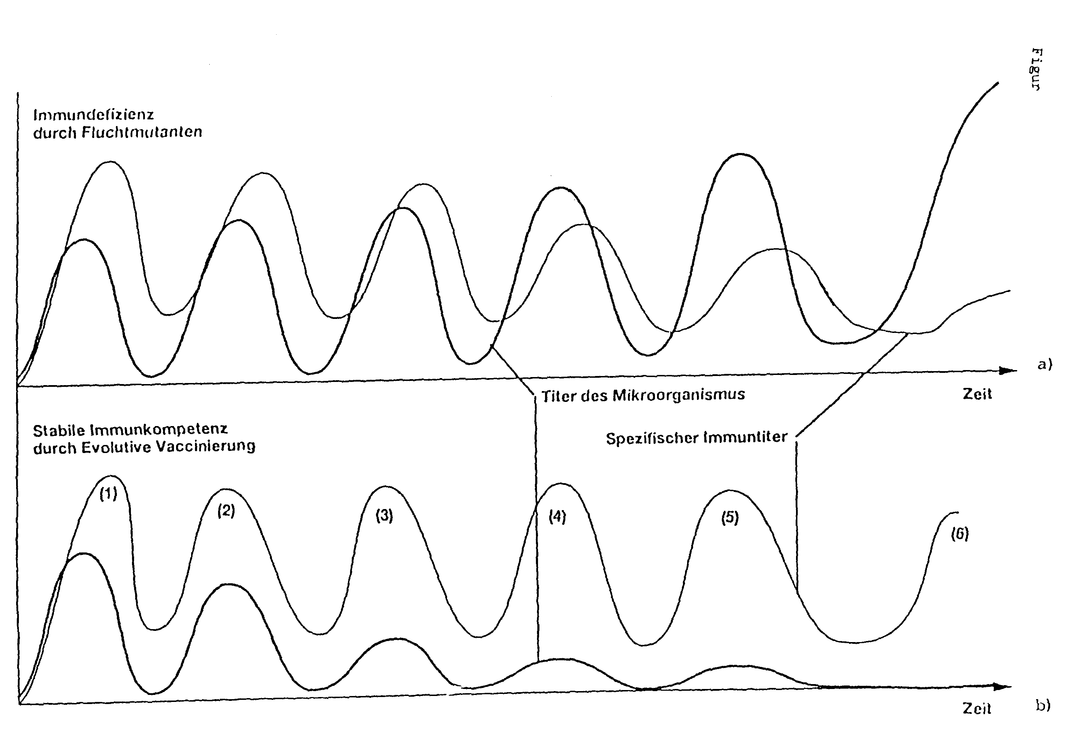

Die Figur zeigt den zyklischen Verlauf infektiöser Erkrankungen. Dabei ist der zyklische Verlauf infektiöser Erkrankungen durch mutierende Mikroorganismen mit immunsupprimierenden Eigenschaften in der oberen Abbildung (a) schematisch dargestellt. Nach anfänglichem Titeranstieg sorgt das zeitversetzt aktivierte Immunsystem für eine Reduktion der infektiösen Erreger. Dennoch führt deren verbleibende Replikation zu neuen Mutanten, aus denen immunresistente Varianten selektiert werden, die neuerlich eine Immunreaktion hervorrufen. Dieser Prozess setzt sich wie im Falle von HIV solange fort, bis die Kapazitäten des Immunsystems erschöpft sind. Dies mag sowohl qualitative als auch quantitative Ursachen haben, die auf einer allgemeinen Schwächung und Schädigung des Immunsystems beruhen. Ist der Erschöpfungszustand erreicht, kann sich die Infektion ungehindert ausbreiten, bis zur vollständigen Inaktivierung des Immunsystems mit den tödlichen Folgen der letalen opportunistischen Erkrankungen.

In der in Abbildung (b) gezeigten evolutiven Vakzinierung kommt es zu einer zeitlich unmittelbaren Aktivierung des Immunsystems, sobald neue Fluchtvarianten auftreten. Dies kann dadurch erfolgen, daß entweder bereits ein passiver Schutz darin besteht, daß entsprechende, vorzugsweise inaktivierende Antikörper oder immunkompetente Zellen vorliegen, oder daß entsprechende Gedächtnis-T-Zellen vorliegen, die direkt eine Aktivierung des humoralen Systems bewirken. In der Konsequenz ist das Immunsystem nahezu zeitgleich befähigt die Fluchtmutanten anzugreifen, wodurch sich deren Möglichkeit zu effizienter Vermehrung stark reduziert. Nach einem oder mehreren Zyklen kommt es zur Eliminierung jeglicher Vermehrung des infektiösen Mikroorganismus.

Die Immunaktivität (1) - Figur b) stellt die erste Antwort des Immunsystems auf die Virusinfektion dar, der Varianten entstammen, die durch die erste Antwort nicht neutralisiert werden können. Sie lösen wie im obigen Beispiel die nächste Immunreaktion (2) aus. Wenn jedoch im vorhergehenden Schritt eine externe Therapie mit einem Wirkstoff durchgeführt wurde, so ergeben sich neben den nicht vorhersehbaren Varianten, die der ersten Immunantwort entkommen, auch solche die erfindungsgemäß vorhersehbar sind. Es sind diejenigen, die gleichzeitig die charakteristischen Mutationen tragen, die für eine Wirkstoffresistenz verantwortlich sind. Wenn diese Varianten jedoch z.B dem Immunsystem bereits vor einem Virusreplikationsbedingten Auftreten präsentiert wurden, z.B. über ein immunogenes Peptid entsprechender Erkennungsregion, dann sind sowohl bereits geringe Konzentrationen entsprechender humoraler Antikörper vorhanden, als auch die zugehörigen Gedächtniszellen. Auf diese Weise ist die spezifische Immunabwehr bereits aktiviert, sobald die Varianten gebildet werden.

Entsprechendes findet im nächsten Zyklus (3) statt, sofern beispielsweise eine Vakzinierung die dritte Fluchtmutante eingeschlossen hat. Die Anzahl notwendiger Zyklen ist von der Art des Therapeutikums und der Art des infektiösen Mikroorganismus abhängig.

## Patentansprüche

1. Substanz oder Substanzgemisch erhältlich durch
a) Züchtung von pathogenen Organismen einer Sorte in Gegenwart eines Wirkstoffes gegen diese pathogenen Organismen,
b) bis zur Bildung von gegen diesen Wirkstoff resistenten pathogenen Organismen (Fluchtmutanten) und
c) Herstellung von Antiseren, in an sich bekannter Weise, gegen diese resistenten pathogenen Organismen (Fluchtmutanten) oder Epitope funktionaler Elemente der Fluchtmutanten.

2. Substanz oder Substanzgemisch nach Anspruch 1 erhältlich durch mehrmaliges Durchlaufen der Verfahrensschritte a) bis c), wobei die im Schritt c) gewonnenen Antiseren entweder als Wirkstoff für Schritt a) verwendet werden oder ein gegen die Fluchtmutanten wirksamer, weiterer Wirkstoff in Schritt a) eingesetzt wird.

3. Substanz oder Substanzgemisch nach Anspruch 1 oder 2, wobei die Züchtung der pathogenen Organismen in Zellen in einem Flußreaktor erfolgt und das Auftreten der Fluchtmutanten der pathogenen Organismen durch Messung der lytischen Aktivität oder der Vermehrung der pathogenen Organismen erfolgt.

4. Substanz oder Substanzgemisch nach Anspruch 1 und/oder 2, wobei die Züchtung der pathogenen Organismen durch Wachstum in Gegenwart steigender Konzentrationen des Wirkstoffes gegen die pathogenen Organismen erfolgt bis die ersten Mutanten auftreten.

5. Substanz oder Substanzgemisch nach mindestens einem der Ansprüche 1 bis 4, wobei die pathogenen Organismen, Viren oder Bakterien, Pilze oder andere Krankheitserreger sind.

6. Substanz oder Substanzgemisch nach mindestens einem der Ansprüche 1 bis 5, wobei die Herstellung von Antiseren mit Epitopen funktionaler Elemente des resistenten pathogenen Organismus erfolgt.

7. Screening-Verfahren zur Identifizierung von wirksamen Substanzen gegen pathogene Organismen, wobei zunächst die pathogenen Organismen in Gegenwart eines Wirkstoffes gezüchtet werden bis zur Bildung von Fluchtmutanten und die Fluchtmutanten dann als Zielorganismen für die Behandlung mit weiteren Substanzen, die von dem ersten Wirkstoff verschieden sein müssen, verwendet werden und die gegen die Fluchtmutanten wirksamen Substanzen als geeignete Wirkstoffe identifiziert werden.

8. Arzneimittel enthaltend eine wirksame Menge eines Wirkstoffes gegen pathogene Organismen und eine Substanz oder ein Substanzgemisch nach mindestens einem der Ansprüche 1 bis 6 sowie übliche pharmazeutische Hilfs- und Trägerstoffe.

## Claims

1. A substance or mixture of substances obtainable by
a) cultivating pathogenic organisms of a particular type in the presence of an active substance against said pathogenic organisms
b) until formation of pathogenic organisms which are resistant to said active substance (escape mutants), and
c) preparing antisera in a per se known manner against said resistant pathogenic organisms (escape mutants) or epitopes of functional elements of said escape mutants.

2. The substance or mixture of substances according to claim 1 obtainable by repeated passing through process steps a) through c) wherein either the antisera obtained in step c) are used as the active substance for step a), or another active substance effective against the escape mutants is employed in step a).

3. The substance or mixture of substances according to claim 1 or 2, wherein the cultivation of said pathogenic organisms is performed in cells within a flow reactor and the occurrence of escape mutants of said pathogenic organisms is detected by measuring the lytic activity or proliferation of said pathogenic organisms.

4. The substance or mixture of substances according to claim 1 and/or 2, wherein the cultivation of said pathogenic organisms is performed by growing them in the presence of increasing concentrations of the active substance against the pathogenic organisms until the first mutants occur.

5. The substance or mixture of substances according to at least one of claims 1 to 4, wherein said pathogenic organisms are viruses or bacteria, fungi, or other pathogens.

6. The substance or mixture of substances according to at least one of claims 1 to 5, wherein said preparation of antisera is performed with epitopes of functional elements of the resistant pathogenic organism.

7. A screening method for identifying active substances against pathogenic organisms wherein said pathogenic organisms are first cultivated in the presence of an active substance until escape mutants occur and said escape mutants are then used as target organisms for the treatment with other substances which must be different from the first active substance, and the substances effective against the escape mutants are identified as suitable active substances.

8. A medicament containing an effective amount of an active substance against pathogenic organisms and a substance or mixture of substances according to at least one of claims 1 to 6 as well as conventional pharmaceutic adjuvants and vehicles.

## Revendications

1. Substance ou mélange de substances, que l'on peut obtenir :
(a) par culture d'organismes pathogènes d'un certain type, en présence d'un principe actif contre ces organismes pathogènes,
(b) jusqu'à la formation d'organismes pathogènes résistant à ce principe actif (mutants fuyants), et
(c) préparation d'antisérums, d'une manière connue en soi, contre ces organismes pathogènes résistants (mutants fuyants), ou contre des épitopes d'éléments fonctionnels des mutants fuyants.

2. Substance ou mélange de substances selon la revendication 1, pouvant être obtenue par un passage à plusieurs reprises par les étapes (a) à (c), les antisérums obtenus dans l'étape (c) étant utilisés comme principe actif pour l'étape (a), ou encore on utilise dans l'étape (a) un autre principe actif, qui agit contre les mutants fuyants.

3. Substance ou mélange de substances selon la revendication 1 ou 2, où la culture des organismes pathogènes s'effectue dans des cellules dans un réacteur de passage, et l'apparition des mutants fuyants des organismes pathogènes est déterminée par une mesure de l'activité lytique ou de la prolifération des organismes pathogènes.

4. Substance ou mélange de substances selon la revendication 1 et/ou 2, où la culture des organismes pathogènes s'effectue par prolifération en présence de concentrations croissantes du principe actif contre les organismes pathogènes, jusqu'à ce qu'apparaissent les premiers mutants.

5. Substance ou mélange de substances selon au moins l'une des revendications 1 à 4, dans lequel les organismes pathogènes sont des virus ou des bactéries, des champignons ou d'autres microorganismes pathogènes.

6. Substance ou mélange de substances selon au moins l'une des revendications 1 à 5, où la préparation d'antisérums a lieu avec des épitopes d'éléments fonctionnels de l'organisme pathogène résistant.

7. Procédé de sélection destiné à identifier des substances actives contre des organismes pathogènes, dans lequel les organismes pathogènes sont d'abord cultivés en présence d'un principe actif jusqu'à formation de mutants fuyants, puis les mutants fuyants sont utilisés comme organismes cibles pour le traitement avec d'autres substances, qui doivent être différentes du premier principe actif, puis on identifie comme étant des principes actifs appropriés les substances actives contre les mutants fuyants.

8. Médicament contenant une quantité efficace d'un principe actif contre des organismes pathogènes et une substance ou un mélange de substances selon au moins l'une des revendications 1 à 6, ainsi que des adjuvants et excipients pharmaceutiques usuels.
